# EUROPÄISCHE PATENTSCHRIFT

(11) **EP 2 867 203 B1**
(45) Veröffentlichungstag und Bekanntmachung des Hinweises auf die Patenterteilung: **28.09.2016**
(21) Anmeldenummer: 13728200.0
(22) Anmeldetag: 13.06.2013
(51) Int. Cl.: C07C 319/18, C07C 323/22, C07C 45/28

(54) **INTEGRIERTES VERFAHREN ZUR HERSTELLUNG VON ACROLEIN UND 3-METHYLMERCAPTOPROPIONALDEHYD**
INTEGRATED METHOD FOR PRODUCING ACROLEIN AND 3-METHYLMERCAPTO PROPIONALDEHYDE
PROCÉDÉ INTÉGRÉ DE FABRICATION D'ACROLÉINE ET DE 3-MÉTHYLMERCAPTOPROPIONALDÉHYDE

(30) Priorität: 27.06.2012 EP 12173892
(43) Veröffentlichungstag der Anmeldung: 06.05.2015
(73) Patentinhaber: Evonik Degussa GmbH, 45128 Essen (DE)
(72) Erfinder: ZACCHI, Pablo, 36608 Mobile, AL (US); FINKELDEI, Caspar-Heinrich, 63755 Alzenau (DE); KÖRFER, Martin, 63796 Kahl (DE)
(86) Internationale Anmeldenummer: PCT/EP2013/062206
(87) Internationale Veröffentlichungsnummer: WO 2014/001093

(56) Entgegenhaltungen:
- EP-A1- 0 022 697
- WO-A1-97/00858
- WO-A1-97/36848
- WO-A1-03/068721
- US-A- 2 626 282
- US-A- 4 225 516
- US-B1- 6 187 963

## Beschreibung

4-Thiapentanal (3-(Methylthio)propionaldehyd, 3-Methylmercaptopropionaldehyd), hier als MMP bezeichnet, ist eine Substanz, die hauptsächlich als ein Ausgangsmaterial für die chemische Synthese der Aminosäure D,L-Methionin oder dem sogenannten Methioninhydroxyanalog (2-Hydroxy-4-(methylthio)buttersäure) eingesetzt wird. Der gängige Weg für die Herstellung von MMP ist die Reaktion zwischen Methylmercaptan (MC) und Acrolein (AC).
Der grundlegende Acrolein-Prozess, der auf der partiellen Oxidation von Propylen basiert, ist aus der Literatur bekannt (siehe Arntz, D., Fischer, A., Höpp, M., Jacobi, S., Sauer, J., Ohara, T., Sato, T., Shimizu, N., Schwind, H., Acrolein and Methacrolein, Ullmann's Encyclopedia of Industrial Chemistry, Wiley-VCH Verlag, 2007, v.a. Seite 7 - 9) und besteht hauptsächlich aus einem Reaktionsschritt, einem Quench-/Nebenproduktabtrennungs-Schritt und einem Absorptions-/Destillations-Schritt. Um das Produkt weiter aufzureinigen (zum Beispiel die Entfernung von flüchtigen Verbindungen, wie Acetaldehyd), können ein oder mehrere extra Destillationsschritt/- schritte angewandt werden. Die Partialoxidationsreaktion wird allgemein in einem salzgekühlten Festbettreaktor bei Betriebstemperaturen von 300 bis 400°C durchgeführt. Der Reaktor ist mit Röhren ausgestattet, die mit einem Partikelkatalysator gefüllt sind, und wird durch Zirkulieren von flüssigem Salz auf den erforderlichen Temperaturen gehalten, welches später in einem Wärmetauscher allgemein durch Erzeugen von Dampf abgekühlt wird. Die Zuführströme Propylen und Luft werden normalerweise mit inerten Verdünnungsgasen wie Stickstoff, Wasserdampf, Kohlendioxid oder Mischungen davon, verdünnt. Auch Kohlenwasserstoffe, welche bei den normalen Reaktionsbedingungen keine nennenswerte Reaktion auf dem Katalysator zeigen, wie der gesättigte Kohlenwasserstoff Propan, können ebenfalls ein Teil des Gemisches sein. Die Verdünnung des Gemisches wird durchgeführt, um die Peak-Temperaturen in der Katalysatorschüttung zu mäßigen und um das Risiko der Bildung von explosiven Gemischen zu minimieren. Der Reaktor ist normalerweise mit einer Nachkühlstufe (das Kühlmedium ist allgemein ein flüssiges Salz) ausgestattet, in dem die Temperatur der gasförmigen Mischung auf 200 - 280°C herabgesetzt wird, bevor diese in die Quenchstufe der ersten Kolonne eintritt. In diesem nachfolgenden Verfahrensschritt wird die Mischung mit Wasser in Kontakt gebracht, um eine schnelle Temperaturverringerung zu erreichen. An dieser Stelle findet auch die Kondensation des während der Oxidationsreaktion erzeugten Wassers sowie des Wassers, das (eventuell) als Verdünnungsmittel dem Reaktionszufuhrgemisch zugesetzt wird, zum größten Teil statt. Nach der Quenchzone der Kolonne strömt das Acrolein-reiche Gas in der Kolonne nach oben und kommt mit einem herabfallenden Wasserstrom in Kontakt, welcher die Aufgabe hat, unerwünschte Nebenprodukte, hauptsächlich Acryl- und Essigsäure und andere Verunreinigungen, zu entfernen. Der herabfallende Wasserfilm stammt aus der Kondensation von Wasser im oberen Teil der Kolonne, die bei Temperaturen von 8 bis 25°C, vorzugsweise von 10 bis 20°C, betrieben wird. Gegebenfalls können zusätzliche Wasserströme dem oberen Teil der Kolonne zugeführt werden, um ein günstigeres Flüssigkeit-zu-Gas-Verhältnis zu erzielen. Die abgetrennten Nebenprodukte verlassen die Kolonne zusammen mit dem kondensierten Wasser über den Sumpf und werden durch thermische oder biologische Mittel entgiftet, nachdem sie die Strippkolonne durchlaufen haben, um den Acroleingehalt dieses Stroms so weit wie möglich zu reduzieren. Das Acrolein wird von dem Gasstrom, welcher den oberen Teil der ersten Kolonne verlässt, in einer nachfolgenden Prozessierungsstufe durch Absorption in einem geeigneten Medium (normalerweise eine wässrige Lösung) abgetrennt. Die erhaltene Flüssigkeit am Boden der Gerätschaft, allgemein einer Absorptionskolonne, wird in eine Destillationskolonne eingespeist, in welcher das niedrigsiedende Acrolein von dem hochsiedenden Absorptionsmedium abgetrennt wird und in flüssiger Form rückgewonnen wird. Wenn Wasser als Absorptionsmedium verwendet wird, wird ein Produkt dicht an der azeotropen Konzentration als Kopfprodukt erhalten. Die Hauptverunreinigung, die vorhanden ist, ist Acetaldehyd, während andere Reaktionsnebenprodukte in sehr geringen oder in Spurenmengen zu finden sind. Das Acrolein kann in dieser Form zu Lagertanks gefördert werden oder weiter aufgearbeitet werden, um seine Reinheit durch Absenken des Gehalts der begleitenden Nebenprodukte zu erhöhen. Angesichts von dessen niedrigem Sauerstoffgehalt kann das nicht kondensierbare Acrolein-arme Gas, das den Absorptionsschritt durch den Oberteil der Kolonne verlässt, zumindest teilweise in den Reaktor als Quelle von inertem Material recycliert werden. Das verbleibende Acrolein-arme Gas wird normalerweise zu einer Verbrennungseinheit für dessen Entsorgung bzw. Entgiftung geleitet. Die partielle Oxidationsreaktion wird allgemein nicht bis zur völligen Umwandlung der Propylen-Zufuhr durchgeführt, um die höchstmöglichen Acrolein-Ausbeuten zu erzielen. Das in der Reaktionstufe nicht umgesetzte Propylen durchläuft den Quench-/Nebenproduktabtrennungs-Schritt und verlässt den Absorber über Kopf zusammen mit den anderen nicht-kondensierbaren Gasen. Durch die Verwendung einer Fraktion dieser Acrolein-armen Gase als Verdünnungsmedium für die Reaktionsmischung wird auch der zusätzliche positive Effekt erzielt, eine Fraktion des nicht umgewandelten Propylen zur Reaktionsstufe zurückzufahren. Auf diese Weise nimmt die Gesamtumwandlung dieses Rohmaterials bei den geeignetsten Reaktionsbedingungen zu, was zu einer höheren Acrolein-Gesamtausbeute führt.
Acrolein ist eine sehr toxische, hoch entflammbare, sehr reaktive Substanz, die eine große Neigung zu hoch exothermen Polymerisationsreaktionen aufweist. Aus diesem letztgenannten Grund wird ein Stabilisator gegen Radikalpolymerisation in mehreren Stufen in dem Prozess und vor der Lagerung beigemischt.
Um hauptsächlich die Sicherheitsrisiken in Zusammenhang mit der Lagerung von Acrolein zu reduzieren, sind mehrere alternative Produktions-/Reinigungskonzepte vorgeschlagen worden. Da die Hauptnutzung von Acrolein die Produktion von MMP ist, beinhalten die Konzepte allgemein die Reaktion von Acrolein zu MMP ohne eine nennenswerte Zwischenspeicherung. Zum Beispiel beschreibt die US 7531066 einen Prozess ähnlich dem oben beschriebenen standardmäßigen, mit der Ausnahme, dass an Stelle der Gewinnung von Acrolein in flüssiger Form als Kopfprodukt des Destillationsschritts eine partielle Kondensation durchgeführt wird und das verbleibende gasförmige Acrolein direkt in einer weiteren Stufe mit flüssigem oder gasförmigem Methylmercaptan in Gegenwart eines Katalysators zu MMP umgesetzt wird.

Die US 5352837 (bzw. WO94/29254) und US 5744647 beschreiben einen Prozess für die Produktion von MMP, in dem Acrolein zuerst durch die partielle Oxidation von Propylen oder Propan in einer Reaktionseinheit produziert wird, die Reaktionsgase danach abgekühlt werden, um Wasser und Nebenprodukte abzutrennen, und der verbleibende Gasstrom, der hauptsächlich nicht kondensierbare Bestandteile und Acrolein enthält, in dem nachfolgenden Prozessierungsschritt mit flüssigem MMP kontaktiert wird, um das Acrolein in der Flüssigphase zurückzubehalten und in diesem gleichen Medium das Acrolein mit Methylmercaptan in Gegenwart eines Katalysators zur Bildung von MMP umzusetzen. Verglichen mit dem herkömmlichen Acrolein-Prozess bietet das in der US 5352837 und US 5744647 beschriebene Verfahren den Vorteil, dass flüssiges Acrolein nicht isoliert und zwischengelagert werden muss. Allerdings ist das Verfahren dadurch gekennzeichnet, dass es keine partielle Rückführung der sauerstoff-armen Gase, die den Acrolein-Absorptionsschritt verlassen, besitzt. Das erforderliche inerte Material zum Verdünnen des Reaktionsgemisches, bevor es in den Reaktor eintritt, ist in diesem Fall Wasserdampf (Dampf). Diese großen Mengen an Wasserdampf, die in den Reaktor eingespeist werden, werden in dem Quenchschritt kondensiert und verlassen den Prozess zusammen mit den Säurenebenprodukten (hauptsächlich Acryl- und Essigsäure). Verglichen mit dem oben beschriebenen herkömmlichen Acrolein-Prozess bringt dieser Prozess den Nachteil mit sich, dass er wesentlich höhere Abwasserbehandlungs-/Entsorgungskosten hat. Darüber hinaus sind die gesamten Acrolein-Ausbeuten, basierend auf dem zugeführten Kohlenwasserstoff zu dem Prozess, allgemein niedriger im Vergleich zu dem Standardverfahren. Wie bereits erwähnt, wird die Reaktion zur Erzielung guter Acrolein-Ausbeuten normalerweise nicht bis zur vollen Umwandlung des zugeführten Propylen betrieben. Höhere Propylen-Umwandlungsraten als der für den eingesetzten Katalysator optimale Bereich ergeben höhere Anteile an Nebenprodukten. Wie bereits erwähnt, wird im konventionellen Acroleinproduktionsverfahren ein Anteil des nicht umgesetzten Propylen als Bestandteil der sauerstoffarmen Gase zwecks der Verdünnung der Feedgasmischung in den Reaktorschritt zurückgeführt. Die Rückführung einer Fraktion des zugeführten Kohlenwasserstoffs zurück zum Reaktor mit dem Rückführgasstrom ermöglicht einen Betrieb nahe an der optimalen Single-pass- bzw. Einmaldurchlauf-Umwandlung, um die Acrolein-Ausbeute zu maximieren, während gleichzeitig die gesamte Kohlenwasserstoffumwandlung von diesem teuren Ausgangsstoff im Vergleich zu einer Single-pass-Einheit erhöht wird. Ein Mangel dieses Recyclingstroms bedeutet mit anderen Worten, dass der in der US 5352837 (bzw. WO94/29254) und US 5744647 beschriebene Prozess eine niedrigere Ausgangsstoffeffizienz besitzt (weniger Acrolein - oder MMP - pro zugeführte Kohlenwasserstoffeinheit) als der herkömmliche Acrolein-Produktionsprozess. Der Einsatz von Wasserdampf als inerte Gasquelle in den zuletzt beschriebenen Prozessen dürfte aus der Abneigung herrühren, das sauerstoffarme Gas, das die Absorptionsstufe verlässt, zurück zum Reaktor recyclieren, da dieses Gas bestimmte Mengen an schwefelhaltigen Verbindungen enthält, die den heterogenen Acrolein-Katalysator negativ beeinflussen würden, sich im System anhäufen können oder unerwünschte Nebenprodukte bilden können, was erhebliche Nachteile mit sich bringen würde.

Die US4225516 bzw. DE2627430 beschreibt ein Verfahren für die Herstellung von MMP, in dem den Beispielen gemäß ein Reaktionsgas, das 48,2 Mol-% Wasser, 41,6 Mol-% N₂, 5,55 Mol-% Acrolein und 0,65 Mol-% Acrylsäure enthält, in eine Acrylsäure-Entfernungseinheit eingespeist wird, später auf etwa 0°C abgekühlt wird, um Wasser zu entfernen und dann durch eine Absorptionseinheit zu laufen, in der das Acrolein in MMP absorbiert wird. Das MMP tritt in den Oberteil der Kolonne bei Temperaturen von etwa -10°C ein. Das im Sumpf der Kolonne erhaltene Gemisch von MMP und Acrolein wird durch einen Reaktor geleitet, in dem das Acrolein mit Methylmercaptan in Gegenwart eines Katalysators reagiert. Methylmercaptan wird in diesem Prozess kontinuierlich dem Reaktor hinzugefügt. Die Gase, welche die Absorptionseinheit verlassen, werden in die Verbrennungseinheit eingespeist. Das Vorhandensein von großen Mengen Wasser in dem Reaktionsgasgemisch, das in die Reinigungsstufe des Prozesses eintritt, deutet darauf hin, dass die Quelle von inertem Material für die Acrolein-Reaktion Wasserdampf ist. Ebenso wie bei dem in der US 5352837 und
US 5744647 beschriebenen Verfahren führt die hohe Menge an Wasser, die in den Reaktor eingespeist wird, im Vergleich mit dem standardmäßigen Acrolein-Produktionsprozess zu größeren Mengen an Abwasser und somit zu höheren Behandlungs-/Entsorgungskosten. Außerdem ist, da kein nicht umgewandeltes Propylen zum Reaktor rückgeführt wird, die gesamte Acrolein-Ausbeute auf Basis des zugeführten Kohlenwasserstoffs allgemein niedriger als in dem Standardprozess. Dies führt zu einem höheren spezifischen Verbrauch des zugeführten Kohlenwasserstoffs (Propylen/Propan) pro erzeugtem Mol Acrolein (oder MMP), was ein großer Nachteil dieser Verfahren ist.

DE-102010064250.9 beschreibt ein Verfahren für die Produktion von MMP, in dem Acrolein, welches durch die partielle Oxidation von Propylen der Gasphase erzeugt wird, zuerst einen Quench-/Nebenprodukt-Abtrennungsschritt durchläuft, dann in MMP absorbiert wird und mit freiem Methylmercaptan oder mit aus dem als Zwischenprodukt gebildeten 3-Methylmercaptopropionaldehyd/Methylmercaptan-Hemithioacetal (=MMP/MC-Hemithioacetal, aus MC+MMP) freigesetzten Methylmercaptan zu MMP reagiert. Diese Erfindung macht von Methylmercaptan, das relativ hohe Verunreinigungen (Dimethylsulfid, Dimethylether) enthält, und von einem homogenen oder heterogenen Katalysator für die MMP-Reaktion Gebrauch. In diesem Prozess besteht das in den Reaktor eingespeiste inerte Material aus einem Gemisch aus Stickstoff, Kohlendioxid und kleinen Mengen an Wasserdampf. Verglichen mit den Prozessen unter Verwendung von Wasserdampf als Hauptquelle von inertem Gas, wie zuvor beschrieben, hat dieser Prozess den Vorteil, dass wesentlich geringere Mengen an flüssigem Abwasser erzeugt werden, aber den Nachteil eines größeren Abgasstroms, welcher ebenfalls schwefelhaltige organische Verbindungen einschließt. Die Menge des Abgasstroms ist auch viel höher im Vergleich zu dem Standard-Acrolein-Prozess. Darüber hinaus, da es keine Rückführung von Propylen zum Reaktionsschritt gibt, weist diese Erfindung eine niedrigere Propylen-Nutzung und damit eine niedrigere Menge an erzeugtem Acrolein (oder MMP) pro Moleinheit zugeführtem Kohlenwasserstoff auf im Vergleich zu dem Standard-Acrolein-Prozess.

**Aufgabe** der vorliegenden Erfindung war es daher, ein integriertes Verfahren zur Herstellung von Acrolein durch katalytische Gasphasenoxidation von Propylen mit sauerstoffhaltigem Gas und Weiterumsetzung des erzeugten Acroleins mit Methylmercaptan zu MMP bereitzustellen, das die Nachteile der bekannten Verfahren möglichst nicht oder nur in veringertem Umfang aufweist.
Insbesondere sollte das erfindungsgemäße Verfahren bei möglichst hohen Acrolein- bzw. MMP-Ausbeuten möglichst energieeffizient arbeiten, also möglichst geringe Energie-bzw. Dampfverbräuche und möglichst geringe Abfallströme aufweisen aber gleichzeitig gewährleisten, Acrolein in möglichst hoher Ausbeute bezogen auf die eingesetzten Mengen an Propylen und in der Folge MMP von möglichst hoher Reinheit und in möglichst hoher Ausbeute herzustellen.

Diese Aufgabe wird erfindungsgemäß **gelöst** durch ein Verfahren zur Herstellung von MMP aus Acrolein und Methylmercaptan gemäß Prozessoption 1, bei dem nacheinander die nachfolgenden Schritte durchgeführt werden
A) Gasphasenoxidation von Propylen mit Hilfe von Luft an einem heterogenen Katalysator in Anwesenheit eines Verdünnungsgases,
B) Auffangen des Acrolein-haltigen Gasstromes aus A) in einer Quenchstufe zur Abtrennung von Nebenprodukten wie z.B. Acrylsäure, Essigsäure, Formaldehyd bzw. deren Folgeprodukte
C) Rückgewinnen von Rest-Acrolein-Anteilen aus der im unteren Teil der Quenchstufe B) vorhandenen Flüssigkeit durch Strippung, insbesondere aus einem dort entnommenen Flüssigkeitsstrom,
D) (nach Teilen des Acrolein-haltigen Gasstromes in mindestens zwei Ströme) Auffangen eines ersten Teiles des Acrolein-haltigen Gasstromes aus der Quenchstufe B) in einer Absorptionsstufe in Gegenwart von Wasser zur Gewinnung einer wässrigen Acrolein-lösung
D1) zumindest teilweise Rückführung des nicht kondensierbaren Gases aus D) als Verdünnungs- bzw. Inertisierungsgas in die Reaktionsstufe A)
E) Destillative Abtrennung des Acroleins aus der wässrigen Acrolein-Lösung aus D) in einer Destillationsstufe
E1) Kondensation des Acrolein-haltigen Destillats aus E) und Zufuhr des Destillats in eine Reaktionsstufe F)
   sowie Zufuhr eines weiteren Teiles des Acrolein-haltigen Gasstromes aus der Quenchstufe B) direkt in die Reaktionsstufe F) zur Umsetzung mit Methylmercaptan in Gegenwart von MMP und/oder MMP/MC-Hemithioacetal.

Ein solches Verfahren (Konfiguration B/Prozessoption 1) hat gegenüber den herkömmlichen Standardverfahren (Konfiguration A) v.a. den Vorteil, dass es bei in etwa den gleichen Acrolein-Ausbeuten und in etwa gleicher Menge an Abgasen eine etwas geringere Menge an Abwasser bei gleichzeitig deutlicher Verringerung des Verbrauchs an demineralisiertem Wasser und eine deutliche Reduzierung des Dampfverbrauchs sowie der Kühlenenergie in Form von Kühlturmwasser oder gekühltem Wasser ermöglicht (Tabelle 1). Darüber hinaus wird die Speicherung bzw. Lagerung von flüssigem Acrolein dabei umgangen, was einen erheblichen Vorteil im Hinblick auf geringere Investionskosten und Vermeidung von Gefahrgutlagerung bietet. Verglichen mit DE102010064250.9 hat diese Erfindung darüber hinaus den Vorteil von höheren Acrolein-Ausbeuten bei weniger Kühlturmwasserverbrauch und wesentlich niedrigeren Abgasmengen und entsprechend wesentlich niedrigeren Abgasbehandlungskosten. Insbesondere ist vorteilhaft, dass das erfindungsgemäße Verfahren einen eigenen Verdünnungs- bzw. Inertisierungsgasstrom aus der Absorptionsstufe D heraus zu erzeugen und zu nutzen ermöglicht und dadurch den Verbrauch an zusätzlichem Inertisierungsgas wie Stickstoff, Dampf oder anderen Inertgasen wie z.B. Methan, Erdgas oder Propan gering zu halten. Inertgase in diesem Zusammenhang sind Gase, die bei den üblichen Produktionsbedingungen am Katalysator nicht reagieren.
Die oben genannte Aufgabe wird erfindungsgemäß auch **gelöst** durch ein Verfahren zur Herstellung von MMP aus Acrolein und Methylmercaptan gemäß Prozessoption 2, bei dem nacheinander die nachfolgenden Schritte durchgeführt werden
A) Gasphasenoxidation von Propylen mit Hilfe von Luft an einem heterogenen Katalysator in einer ersten Reaktionseinheit in Anwesenheit eines Verdünnungsgases sowie
A1)gleichzeitige Gasphasenoxidation von Propylen mit Hilfe von Luft an einem heterogenen Katalysator in Anwesenheit eines Verdünnungsgases in einer weiteren Reaktionseinheit
B) Auffangen des Acrolein-haltigen Gasstromes aus A) in einer Quenchstufe unter Kühlung zur Abtrennung von Nebenprodukten, wie z.B. Acrylsäure, Essigsäure, Formaldehyd bzw. deren Folgeprodukte,
B1)Auffangen des Acrolein-haltigen Gasstromes aus A1) in einer parallelen Quenchstufe zur entsprechenden Abtrennung von Nebenprodukten,
C) Rückgewinnen von Rest-Acrolein-Anteilen aus der im unteren Teil der Quenchstufe B) vorhandenen Flüssigkeit durch Strippung, insbesondere aus einem dort entnommenen Flüssigkeitsstrom,
D) Auffangen zumindest eines Teiles des Acrolein-haltigen Gasstromes aus der Quenchstufe B) in einer Absorptionsstufe in Gegenwart von Wasser zur Gewinnung einer wässrigen Acrolein-lösung
D1)zumindest teilweise Rückführung des nicht kondensierbaren Gases aus D), welches auch noch unumgesetzes Propylen enthält, als Verdünnungs- bzw. Inertisierungsgas in die Reaktionsstufe A) und A1)
E) Destillative Abtrennung des Acroleins aus der wässrigen Acroleinlösung aus D) in einer Destillationsstufe
E1) Kondensation des Acrolein-haltigen Destillats aus E) und Zufuhr des Destillats in eine Reaktionsstufe F)
   sowie Zufuhr des Acrolein-haltigen Gasstromes aus der Quenchstufe B1) direkt in die Reaktionsstufe F) zur Umsetzung mit Methylmercaptan in Gegenwart von MMP und/oder MMP/MC-Hemithioacetal.

Ein solches Verfahren (gemäß Konfiguration C/Prozessoption 2) hat gegenüber den herkömmlichen Standardverfahren (Konfiguration A) analog wie Prozessoption 1 v.a. den Vorteil, dass es bei in etwa den gleichen Acrolein-Ausbeuten und in etwa gleicher Menge an Abgasen eine etwas geringere Menge an Abwasser bei gleichzeitig deutlicher Verringerung des Verbrauchs an demineralisiertem Wasser und eine deutliche Reduzierung des Dampfverbrauchs sowie der Kühlenenergie in Form von gekühltem Wasser ermöglicht (Tabelle 1). Ebenso wird die Speicherung bzw. Lagerung von flüssigem Acrolein dabei umgangen, was einen erheblichen Vorteil im Hinblick auf geringere Investionskosten und Vermeidung von Gefahrgutlagerung bietet. Darüberhinaus bietet sie noch den Vorteil, die Vorzüge des Verfahrens der DE102010064250.9 zu integrieren, insbesondere die schnelle und effiziente direkte Umsetzung des gasförmigen Acroleins mit MC zu MMP und dabei gleichzeitig eine Einsparung beim Stromverbrauch gegenüber DE102010064250.9 (Prozesskonfiguration D).
Verglichen mit DE102010064250.9 hat die erfindungsgemäße Prozessoption 2 analog zu Prozessoption 1 auch noch den Vorteil von höheren Acrolein-Ausbeuten und wesentlich niedrigeren Abgasmengen und entsprechend niedrigeren Abgasbehandlungskosten. Ebenso ist vorteilhaft, dass das erfindungsgemäße Verfahren einen eigenen Verdünnungs- bzw. Inertisierungsgasstrom aus der Absorptionsstufe D heraus zu erzeugen und zu nutzen ermöglicht und dadurch den Verbrauch an zusätzlichem Inertisierungsgas wie Stickstoff, Dampf oder anderen Inertgasen wie z.B. Methan, Erdgas oder Propan gering zu halten.
Bevorzugt wird ein Verfahren dadurch gekennzeichnet, dass jeweils Schritt A) bzw. Schritt A1) in einem Rohrbündelreaktor durchgeführt werden, dessen Rohre den Katalysator beinhalten. Im Rohrbündelreaktor erfolgt dann die partielle Oxidationsreaktion zu Acrolein. An Stelle von einem einzigen Rohrbündelreaktor können auch mehrere Reaktoren parallel betrieben werden. Dies ermöglicht eine maximale Raum-/Zeitausbeute.
Bevorzugt wird dabei ein Salzbad zur Kühlung des Rohrbündelreaktors verwendet, da hier die Temperatur sehr zuverlässig kontrolliert werden kann. Das Salzbad wird vorzugsweise auf einer Temperatur von 300 bis 400°C gehalten, um bestmöglichen Umsatz und Selektivität zu gewährleisten. Der sich dabei einstellende Druck reicht typischerweise von 1,3 bis 3 bara. Die zugeführten Gase Luft, Verdünnungsgas Propylen und Dampf müssen daher durch eine Kompressionstufe vorher auf das erforderliche Druckniveau gebracht werden.
Außerdem wird das Verfahren bevorzugt so geführt, dass der Acrolein-haltige Gasstrom aus A) bzw. aus A1) mit einer Temperatur von jeweils 200-280°C in den entsprechenden Schritt B)bzw. B1) gelangt. Die erforderliche Abkühlung des Gases geschieht vorteilhaft mit Hilfe einer Nachkühlstufe.
Bevorzugt wird im oberen Drittel der jeweiligen Quenchkolonne B bzw. B1 ein Teilstrom des dort jeweils vorhandenen Kondensats abgeführt und gegebenenfalls nach Abkühlen, vorzugsweise auf < 20°C, dem Kopf der jeweiligen Kolonne B bzw. B1 wieder zugeführt (oberes Umpumpen). Auf dem Weg zum Oberteil der Kolonne wird das Reaktionsgas mit einem im Gegenstrom fließenden Wasserstrom in Kontakt gebracht, welcher die Menge an Nebenprodukten des Gasstroms weiter reduziert. Der Wasserstrom stammt aus der Kondensation, die während des weiteren Kühlens des Reaktionsgases auf < 20°C im oberen Abschnitt der Kolonne vonstatten geht (oberes Umpumpen).

Weiter bevorzugt wird im Sumpf der jeweiligen Quenchkolonne B bzw. B1 ein Teilstrom der dort jeweils kondensierten Flüssigkeit abgeführt und nach Abkühlen dem unteren Drittel der jeweiligen Kolonne B bzw. B1 wieder zugeführt wird (unteres Umpumpen). Eine große Fraktion der Nebenprodukte, hauptsächlich Acryl- und Essigsäure, werden in der kondensierten Flüssigkeit zurückbehalten und verlassen dabei die Quenchkolonne durch den Sumpf. Durch das gleichzeitige Zirkulieren der Flüssigkeit mittels eines Umpumpsystems und externe Kühlung wird die Flüssigkeit gleichzeitig als Kühlmedium zum Quenchen des Reaktionsgases genutzt (unteres Umpumpen). Der Flüssigkeitsstrom, der dabei die Quench-Kolonne verlässt, wird vorzugsweise zum Oberteil einer Stripperkolonne (C) gepumpt, in welcher eine große Fraktion des gelösten restlichen Acroleins zurückgewonnen wird. Die restliche Flüssigkeit wird dann z.B. in eine thermischen Oxidationsstufe oder eine biologische Behandlungseinheit zur Entsorgung eingespeist.
Zur Erhöhung der Ausbeute werden vorzugsweise die rückgewonnenen Rest-Acrolein-Anteile aus Schritt C) in Schritt B) zurückgeführt.
Die Absorptionsstufe D wird bevorzugt bei Temperaturen von 1 bis 25°C, besonders bevorzugt von 3 bis 15°C, betrieben um eine möglichst vollständige Absorption des Acroleins zu gewährleisten.
Die Destillationsstufe E wird vorzugsweise bei einem Druck von 0,4 bis 1,2 bara (bar absolut) und bei den sich dabei einstellenden Temperaturen z.B. von typischerweise 25 bis 65 °C am Kolonnenkopf betrieben. Dadurch wird sichergestellt, eine Acrolein-reiche Mischung mit Wasser nahe der azeotropen Zusammensetzung über dem Oberteil der Kolonne und einen nahezu Acrolein-freien Wasserstrom über den Boden der Kolonne zu erhalten.
Desweiteren bevorzugt ist, dass das Acroleinfreie wasserhaltige Sumpfprodukt der Destillation zur Absorptionsstufe D zurückgeführt wird, um dort möglichst wenig zusätzliches Wasser zu benötigen, wobei gleichzeitig die Abwasserfrachten verringert werden.
Für den Reaktionschritt F der 1,4-Addition von Methylmercaptan an Acrolein wird vorzugsweise ein basenhaltiger Katalysator, besonders bevorzugt ein Amin, ganz besonders bevorzugt im Gemisch mit einer Säure verwendet. Dies gewährleistet eine hohe Umsatzrate und eine hohe Selektivität der MMP-Bildung. Der Katalysator wirkt durch seine in der Regel gute Löslichkeit als homogener Katalysator. Dabei wir vorzugsweise ein geringer Überschuss von ca. 1,005 mol Methylmercaptan /mol Acrolein) eingesetzt, der einen hohen Umsatz des Acroleins gewährleistet.
Als Base wird bevorzugt ein ggf. substituiertes N-heterocyclisches Amin oder ein Amin der Formel NR1 R2R3 verwendet, wobei R1, R2 und R3 gleich oder verschieden sind und unabhängig voneinander H, C1- bis C14-Alkyl, oder C7- bis C14-Aralkyl bedeuten, mit der Maßgabe, dass wenn R1, R2 oder R3 gleich H sind die beiden jeweiligen anderen Reste ungleich H sein müssen.
Besonders geeignete Basen sind beispielsweise Pyridin, alkylsubstituiertes Pyridin, vorzugsweise Picolin oder Lutidin, Trimethylamin, Triethylamin, Tripropylamin, Tributylamin, Tridecylamin, Tridodecylamin oder Dimethylbenzylamin.
Besonders geeignete Säuren sind Mineralsäuren, vorzugsweise Salzsäure, Schwefelsäure oder Phosphorsäure oder organische Säuren, vorzugsweise Ameisensäure, Essigsäure, Propionsäure, Milchsäure, Bernsteinsäure, Weinsäure oder Citronensäure.
Die Umsetzung in der Reaktionsstufe F wird vorzugsweise bei einem Druck von 1,0 bis 2,5 bara und bei Temperaturen von 50 bis 100 °C betrieben, vorzugsweise bei 60 bis 90 °C, ganz besonders bevorzugt bei 75 - 85 °C.
Weiter bevorzugt ist, dass der weitere Teil des Acrolein-haltigen Gasstroms aus Quenchstufe B), der direkt in die Reaktionsstufe F) geführt wird, einer Menge von 30 - 70 Gew.-%, vorzugsweise 40-60 Gew.-% und ganz besonders bevorzugt von 45 - 55 Gew.-%, insbesondere con ca. 50 Gew.-% der Gesamtmenge des Acrolein-haltigen Gases aus B) entspricht. Dadurch werden besonders niedrige Verbräuche an Kühlenergie bzw. von Dampf im Vergleich zum Standardproduktionsverfahren (Konfiguration A in Tabelle 1) ermöglicht. Durch die erfindungsgemäße Reaktionsführung insbesondere die gleichzeitige Verwendung flüssiger und gasförmiger Acrolein -Anteile wird eine höhere MMP-Ausbeute als im Verfahren gemäß DE 10 2010 064 250.9 erzielt, was ein weiterer großer Vorteil ist in einem großtechnischen Verfahren.
Besonders bevorzugt ist also auch ein Verfahren gemäß Prozessoption 1, welches dadurch gekennzeichnet ist, dass die Gasphasenoxidation von Propylen in Schritt A parallel in mindestens einem weiteren Reaktor (A1) ausgeführt wird.
Weiter besonders bevorzugt ist auch ein Verfahren gemäß Prozessoption 1, welches dadurch gekennzeichnet ist, dass das Auffangen des Acrolein-haltigen Gasstroms aus A1 zur Abtrennung von Nebenprodukten in einer weiteren Quenchstufe B1 geschieht. Bei dieser Variation verläuft das weitere Verfahren gemäß Prozessoption 2.

**Zusammenfassend stellen sich wichtige Vorteile des erfindungsgemäßen Verfahrens** (Konfiguration B, C, Tab.1) **wie folgt dar:**
- Verglichen mit dem Acrolein/ MMP-Standardprozess (Konfiguration A, Tab.1):
   o keine Lagerung von Acrolein
   o ca. 50 % weniger Dampfverbrauch
   o ca. 50 % weniger Verbrauch an demineralisiertem Wasser
   o geringerer Verbrauch an Kühlenergie
   o ähnliche Abgasmenge, aber jetzt Spuren von schwefelhaltigen Verbindungen enthaltend (gut entsorgbar)
- Verglichen mit MMP / Dampf-Inertisierung (Konfiguration E, Tab.1):
   o um mehr als 50 %geringere Menge an Abwasser
   o fast 40 % geringerer Dampfverbrauch
   o höherere Acrolein-Ausbeute
   o geringere Menge an Abgas (mit schwefelhaltigen Verbindungen)
- Verglichen mit DE 10 2010 064 250.9 (MMP-Kombi, Konfiguration D, Tab.1)
   o ca. 50 % geringere Menge an Abgas (mit schwefelhaltigen Verbindungen, (gut entsorgbar)
   o höherer Dampfverbrauch in dem Prozess
   o höhere Acrolein-Ausbeute
   o geringerer Stromverbrauch
   o keine externe Quelle von Verdünnungs-/Inertisierungsgas erforderlich

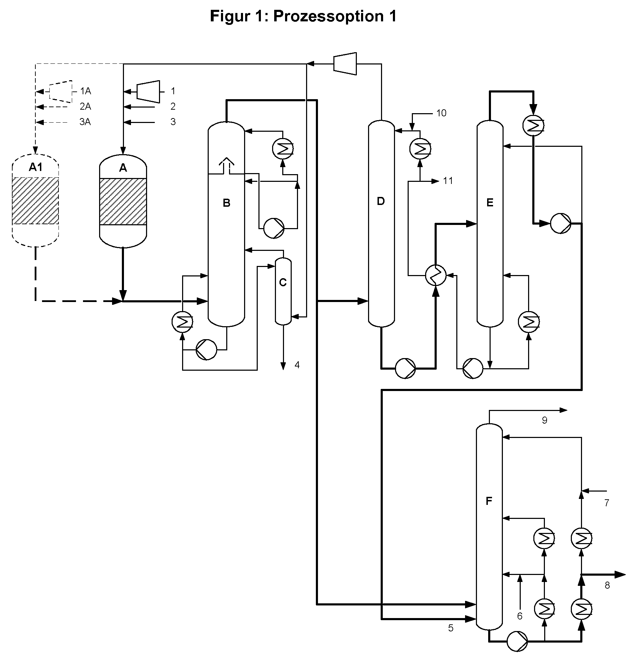

### Beschreibung der Prozessoption 1 gemäß Figur 1

In einer bevorzugten Form der Erfindung (vgl. Figur 1 entsprechend Prozessoption 1) wird der gasförmige Kohlenwasserstoff (3), vorzugsweise Propylen, zusammen mit komprimierter Umgebungsluft (1), geringen Mengen an Wasserdampf (2) und einem sauerstoffarmen Recyclinggas zu einem Rohrbündelreaktor zugeführt, in dem die partielle Oxidationsreaktion zu Acrolein erfolgt. An Stelle von einem Rohrbündelreaktor können auch mehrere Reaktoren parallel betrieben werden, jeweils vorzugsweise mit separaten Propylen-, Luft- und Wasserdampf-Zuführleitungen (hier mit 3A, 1A bzw. 2A bezeichnet). Neben Acrolein werden Nebenprodukte wie Acrylsäure, Essigsäure, Formaldehyd, Acetaldehyd, Kohlendioxid, Kohlenmonoxid und kleine Mengen an anderen Verbindungen in dem Reaktionsschritt gebildet. Die Temperatur des Kühlsalzes wird auf 300 bis 400°C gehalten. Nach der Reaktionszone wird das Gasgemisch vorzugsweise in einem integrierten Nachkühler abgekühlt, welcher wassergekühlt oder bevorzugt salzgekühlt ist, und tritt in die erste Kolonne (Quenchkolonne B) ein. Wie in dem oben beschriebenen herkömmlichen Acrolein-Prozess wird in diesem Anlagenteil das Gasgemisch mit großen Mengen an Wasser kontaktiert, um die heißen Gase weiter abzukühlen. Der größte Teil des in dem Gemisch enthaltenen Wasserdampfs, der hauptsächlich als Nebenprodukt der Oxidationsreaktionen resultiert, wird in dieser Stufe des Prozesses kondensiert. Eine große Fraktion der Nebenprodukte, hauptsächlich Acryl- und Essigsäure, werden in der kondensierten Flüssigkeit zurückbehalten und verlassen die Quenchkolonne B durch den Sumpf. Diese Flüssigkeit wird ebenfalls mittels eines Umpumpsystems zirkuliert und wird so als Kühlmedium zum Quenchen des Reaktionsgases genutzt (unteres Umpumpen). Auf dem Weg zum Oberteil der Kolonne wird das Reaktionsgas mit einem im Gegenstrom fließenden wässrigen Strom in Kontakt gebracht, welcher die Menge an Nebenprodukten des Gasstroms weiter reduziert. Der Wasserstrom stammt aus der Kondensation, die während des weiteren Kühlens des Reaktionsgases auf < 20°C im oberen Abschnitt der Kolonne vonstatten geht (oberes Umpumpen). Der Flüssigkeitsstrom, der die Quench-Kolonne verlässt, wird zum Oberteil einer Stripperkolonne (C) gepumpt, in welcher eine große Fraktion des gelösten Acrolein rückgewonnen wird. Die restliche Flüssigkeit wird dann z.B. in einer thermischen Oxidationsstufe oder eine biologische Behandlungseinheit für ihre Entsorgung eingespeist.
Das Acrolein-reiche Gas aus dem Oberteil der Kolonne (B) wird in zwei Ströme geteilt. Der erste Strom wird in eine Absorptionskolonne (D) eingespeist, in welcher das Acrolein in einem Flüssigkeitsstrom, der hauptsächlich Wasser enthält, absorbiert wird. Die Menge an Gas, die durch diese Kolonne strömt, wird durch die erforderliche Menge an Recyclinggas für den/die Reaktor(en) begrenzt, angesichts der Tatsache, dass das Acrolein-arme Gas, das die Kolonne verlässt, vollständig zu dem/den Reaktor(en) nach einer Kompressionsstufe geschickt wird. Der Acrolein-reiche Wasserstrom im Sumpf der Kolonne D wird in eine Destillationskolonne (E) eingespeist, in der das Acrolein vorzugsweise unter leichten Vakuumbedingungen destilliert wird, um eine einphasige Acrolein-reiche Mischung mit Wasser über den oberen Teil der Kolonne und einen nahezu Acrolein-freien Wasserstrom über den Kolonnensumpf zu erhalten. Dieser Wasserstrom wird anteilig zum Oberteil der Absorptionskolonne (D) zurückgeschickt, um möglichst wenig zusätzliches Wasser zu benötigen wobei gleichzeitig die Abwasserfrachten verringert werden. Der Strom passiert dabei einen Wärmetauscher, über den der Zufuhrstrom zur Detillationskolonne E vorteilhafterweise erwärmt wird, und eine weitere Reihe von Wärmetauschern, um eine Endtemperatur zwischen 0 und 20°C, vorzugsweise zwischen 4 und 12°C, zu erreichen. Der im Oberteil der Destillationskolonne E erhaltene Acrolein-reiche Strom hat vorzugsweise eine Konzentration nahe an der azeotropen Zusammensetzung. Der gasförmige Acrolein-Strom wird kondensiert und teilweise als Rücklauf in der Destillationskolonne genutzt. Das restliche kondensierte Acrolein (5) wird ohne Zwischenspeicherung zum Sumpf des Reaktors F, bevorzugt eine Reaktivabsorptionsstufe in Form einer Reaktivabsorptionskolonne) geschickt, worin eine Reaktion mit Methylmercaptan unter Bildung von MMP erfolgt. In dem Absorption/Destillationskreislauf wird entsalztes Wasser hinzugeführt (10), um die Akkumulation von Reaktionsnebenprodukten in diesem Kreislauf zu vermeiden. Ein Purgestrom (11) aus diesem Kreislauf wird aus dem Strom, der aus der Destillationskolonne E in Richtung Absorptionskolonne D führt, ausgeschleust. Dieser Purgestrom kann teilweise zum oberen Umpumpkreislauf der Quenchkolonne B geführt werden, um das Verhältnis von Flüssigkeit zu Gas in dem mittleren Kolonnenteil zu erhöhen und dadurch eine bessere Nebenproduktabtrennung zu erzielen. Der Purgestrom 11 kann auch direkt in eine thermischen Oxidationsstufe oder eine biologische Behandlungseinheit zur Entsorgung eingespeist werden.
Die andere Fraktion des Acrolein-reichen Gases, das die Kolonne B verlässt, wird ebenfalls in den Reaktor F eingespeist. In diesem Anlagenteil wird das Acrolein zuerst in MMP absorbiert und reagiert dann mit Methylmercaptan (6) in Gegenwart eines homogenen Katalysators (7) unter Bildung von mehr MMP (überschüssiges Methylmercaptan ∼ 1,005 mol/mol) als in dem Verfahren gemäß DE 10 2010 064 250.9. Analog dem in dieser Patentanmeldung beschriebenen System kann hier das produzierte MMP mit einer Pumpe vom Sumpf der Kolonne entnommen und dann in zwei Schritten, zuerst mit Kühlturmwasser (CTW) bis ∼ 35°C und zweitens mit gekühltem Wasser (CW) auf <10°C abgekühlt werden. Das kalte MMP tritt im Oberteil der reaktiven Absorptionskolonne ein und dient als Absorptionsmedium. Entweder nach der ersten oder der zweiten Kühlstufe verlässt eine Fraktion des MMP den Prozess als Produktstrom (8). Eine zweite Umpumpung unter Verwendung von MMP wird in den Mittelabschnitt der Kolonne eingespeist. Die niedrigen Temperaturen, die im Oberteil der reaktiven Absorptionskolonne angewandt werden, tragen zu einer Reduzierung von Acrolein-, Methylmercaptan- und MMP-Verlusten bei. Das Abgas, das die Kolonne verlässt, kann z.B. in einer thermischen Oxidationsstufe entsorgt werden. Vor dem Reaktor tritt der vom Kondensator der Destillationskolonne E kommende gasförmige und/oder flüssige Acrolein-Strom in das System ein.

Alle in Figur 1 dargestellten Wärmetauscher können auch mehrere Wärmetauscher in Reihe oder parallel angeordnet repräsentieren, wobei nicht notwendigerweise die gleichen Kühlmedien verwendet werden.

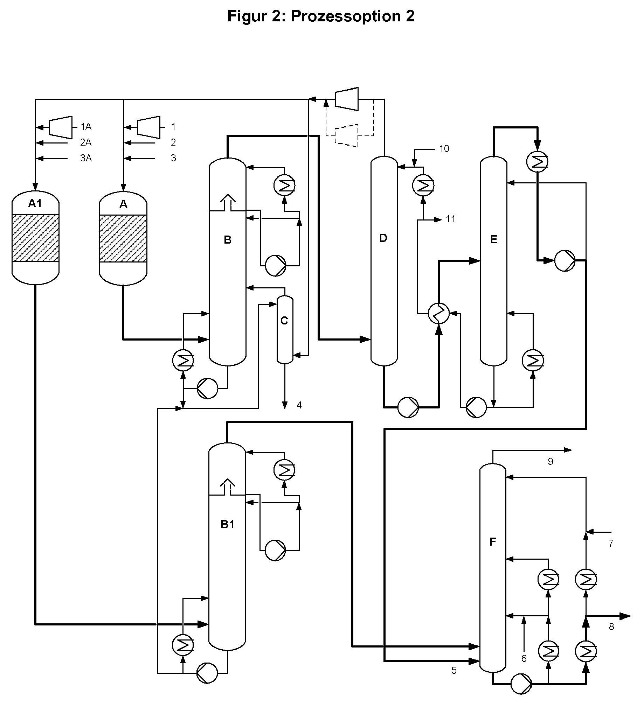

### Beschreibung der Prozessoption 2 gemäß Figur 2

Alternativ wird in einer anderen bevorzugten Form der Erfindung ein Prozess, bestehend aus mindestens zwei separaten Acrolein-Reaktoren, betrieben (Fig. 2). Der gasförmige Kohlenwasserstoff (3), vorzugsweise Propylen, wird zusammen mit komprimierter Umgebungsluft (1), kleinen Mengen an Wasserdampf (2) und einem sauerstoffarmen Recyclingstrom in den ersten Rohrbündelreaktor (A) eingespeist, in dem die partielle Oxidationsreaktion zu Acrolein stattfindet. Wie oben unter Figur 1 beschrieben, werden in der ersten Kolonne (B) die Acrolein-reichen Gase abgekühlt und von einer großen Fraktion der Nebenprodukte befreit. Die größte Fraktion des Acrolein, das in dem die Kolonne B verlassenden Abwasserstrom gelöst ist, wird in der Strippkolonne C zurückgewonnen. Die Gase, welche die Kolonne B verlassen, treten in den Absorber (D) ein, in dem das Acrolein in einem Flüssigkeitsstrom, welcher hauptsächlich Wasser enthält, absorbiert wird. Wie in der Prozessoption 1 der Erfindung wird Acrolein in Kolonne E destilliert und der Flüssigproduktstrom wird sofort ohne eine Zwischenspeicherung in den Reaktor F (Reaktivabsorptionsstufe) eingespeist. Von dem Acrolein- und sauerstoffarmen Gas, das die Kolonne D verlässt, wird nur die Fraktion, die für die Verdünnung der zugeführten Mischung von Reaktor A erforderlich ist, recycliert. Das restliche Gas, das nicht umgesetztes Propylen enthält, wird als Verdünnungs-/Inertisierungsgas in einen zweiten Acrolein-Reaktor (A1) eingespeist, welcher ebenfalls mit einer separaten Luft- und Propylen-Zufuhr versehen ist. Das den Reaktor A1 verlassende Gas tritt in die Kolonne B1 durch den Boden ein. B1 ist analog zu der Kolonne B für den ersten Reaktor aufgebaut. Der die Kolonne B1 verlassende Wasserstrom wird zu der Strippkolonne C geschickt, in der eine große Fraktion des in diesem Strom vorhandenen Acrolein zurückgewonnen wird. Der die Kolonne C verlassende Wasserstrom wird durch thermische oder biologische Mittel entsorgt. Die Gase, welche die Kolonne B1 durch den Kopf verlassen, werden direkt in die Reaktivabsorptionsstufe F eingespeist. In diesem Anlagenteil wird das Acrolein zuerst in MMP absorbiert und reagiert dann mit Methylmercaptan (6) in Gegenwart eines homogenen Katalysators (7) unter Bildung von mehr MMP (überschüssiges Methylmercaptan ∼ 1,005 mol/mol) ähnlich der Ausführungsform, die in DE102010064250.9 beschrieben wird. Der MMP-Produktstrom ist als Strom 8 dargestellt. Die Abgase des Prozesses (9) werden zu einer thermischen Oxidationsstufe geschickt. Analog Prozesskonfiguration 1 wird dem Absorption/Destillationskreislauf entsalztes Wasser hinzugeführt (10), um die Akumulation von Reaktionsnebenprodukte in diesem Kreislauf zu vermeiden. Ein Purgestrom (11) aus diesem Kreislauf wird aus dem Strom, der aus der Destillationskolonne E in Richtung Absorptionskolonne D führt, ausgeschleust. Dieser Purgestrom kann teilweise zum oberen Umpumpkreislauf der Quenchkolonne B bzw. B1 geführt werden, um das Flüssigkeit zu Gas Verhältnis in dem mittleren Kolonnenteil zu erhöhen und dadurch eine bessere Nebenproduktabtrennung zu erzielen. Der Purgestrom 11 kann auch direkt in eine thermische Oxidationsstufe oder eine biologische Behandlungseinheit zur Entsorgung eingespeist werden.

### Vergleich zwischen verschiedenen Prozesskonfigurationen:

Im Folgenden sind die Resultate einer Computersimulation auf Basis des Simulationsprogrammes Aspen Plus (Version 7.1) der Firma Aspen Technology, Inc. dargestellt. Die Simulation basiert auf NRTL-HOC- (Non-Random-Two-Liquid model/ Hayden O'Connel-Gleichung) sowie NRTL-RK- (Non-Random-Two-Liquid model / Redlich-Kwong-Gleichung) Modellen. Die nötigen binären Wechselwirkungsparameter wurden zum Teil aus eigenen Messdaten bzw. aus der in Literatur/Stoffdatenbanken vorhandenen Messdaten abgeschätzt. Darüber hinaus ist die Simulation mit Hilfe von realen Anlagendaten validiert worden.
Für die Simulationsrechnung der Reaktionsstufe wurde eine Acroleinausbeute basierend auf dem Einsatzstoff Propylen von 81,02% in das ASPEN-Programm eingegeben. Der eingegebene Umsatz des Propylen betrug 97,1%, bezogen auf einen Einmaldurchlauf im Verfahren (analog zu Konfiguration D bzw. E).
Die Simulationsergebnisse für mehrere Prozesskonfigurationen hinsichtlich der Acrolein-Gesamtausbeute, der Propylen-Umwandlung, der Menge an Abgas und der flüssigen Abflüsse und die Verbrauchswerte von Dampf und Elektrizität werden verglichen. Die Prozesskonfigurationen werden wie folgt detailliert aufgeführt:
**Konfiguration A (Vergleichsbeispiel 1):** sie entspricht dem beschriebenen herkömmlichen Acrolein-Produktionsprozess (Ullmann's Encyclopedia of Industrial Chemistry, Wiley-VCH Verlag, 2007, v.a. Seite 7 - 9), bestehend aus drei Hauptprozessblöcken: einem Reaktionsschritt, welcher eine Kompressorstufe für Luft, einen Kompressor für Recyclinggas, eine Propylen-Verdampfungseinheit und mindestens einen Rohrbündelreaktor mit integriertem Nachkühler und Dampfgenerator einschließt; einem Prozessblock zum Quenchen und einer Nebenprodukt-Abtrennung, bestehend aus mindestens einer Kolonne, einem Abwasserstripper und der erforderlichen Wärmeaustausch- und Pumpgerätschaft; und einem Absorptions-/Destillations-Prozessschritt, bestehend aus einer Absorptionskolonne, einer Destillationskolonne und der notwendigen Wärmeaustausch- und Pumpgerätschaft. Die größte Fraktion der Absorptionsmedien ist entmineralisiertes Wasser. Das Acrolein wurde als Kopfprodukt der Destillationskolonne mit einer Zusammensetzung nahe an der azeotropen Zusammensetzung (Acrolein/Wasser) zurückgewonnen. Die Hauptverunreinigung, die immer noch in dem Produkt vorhanden war, entspricht Acetaldehyd. Für die Simulationsläufe wurde die in den Reaktor eingespeiste Menge an Dampf auf 0,19 kg/kg zugeführtes Propylen eingestellt. Der Auslassdruck der Frischluft und der Recyclinggaskompressoren wurde mit 2,7 bara gewählt. Die Kompression wurde als isentropisch in der Simulation betrachtet (Isentropische Effizienz: 0,65 / 1,0 Mechanische Effizienz). Die Temperatur der Frischluft am Einstrittspunkt des Kompressors wurde auf 30°C gesetzt. In dieser Konfiguration wird das Acrolein-arme Gas, das die Absorptionseinheit verlässt, teilweise als Recyclinggas wiederverwendet, um das Reaktionsgemisch zu verdünnen (Verdünnungs-bzw. Inertisierungsgas). Die restliche Fraktion von Acrolein-armem Gas verlässt das System und ist hier als "Abgas" gekennzeichnet. Es gibt zwei Flüssigkeitsabflüsse der Anlage: einen, welcher die Strippkolonne des Quench- und Nebenproduktabtrennungs-Schritts verlässt, und einen viel kleineren, welcher einem Purgestrom des Absorptions-/Destillationsschritts entspricht.
**Konfiguration B (Beispiel 1):** Sie entspricht der oben beschriebenen **Prozessoption 1.** In Analogie zu der Konfiguration A wurde der Kompressionsdruck der Gasströme auf 2,7 bara und die Menge an zugeführtem Dampf auf 0,19 kg/kg zugeführtes Propylen eingestellt. Dazu wurde eine Propylenverdampfungseinheit mit betrachtet. Alle anderen Prozessparameter betreffend den Druck und die Temperatur in den Abschnitten A/A1, B, C, D und E (Figur 1) waren identisch mit den in der Konfiguration A gewählten. Die molaren Flüssigkeitsströme, wie jene der Umpumpungen und der Absorptionsflüssigphase, wurden in Abhängigkeit von der Gesamtgasströmung durch die Kolonnen eingestellt. Darüber hinaus waren die Zahl der theoretischen Stufen der Kolonnen und das spezifische Verhältnis von Recyclinggas/zugeführtes Propylen die gleichen wie in der Konfiguration A verwendet.
   Für die Simulationsrechnungen wurden außerdem folgende Ströme eingesetzt: Der Anteil des Stroms aus der Quenchkolonne B in Richtung Absorber/Desorbersystem (Kolonne D) betrug 49,51 Gew.-%, der Anteil zum Reaktivabsorber (Kolonne F) 50,49 Gew.-%.
   Im Anlagenteil Reaktor F wird der Acrolein enthaltende Gasstrom, welcher von der Quenchkolonne B kommt, mit einem Gemisch von MMP, MC, MMP/MC-Hemithioacetal und Wasser kontaktiert. Analog wie in DE102010064250.9 beschrieben, wird das Acrolein in diesem flüssigen Gemisch absorbiert und reagiert in Gegenwart eines Katalysators mit dem freien MC oder mit dem aus der Hemiothiocetal-Form von MC und MMP freigesetzten MC, um mehr MMP aufzubauen. Die Reaktion erfolgt auf den Einbauten ebenso wie in der im Sumpf der Kolonne zurückgehaltenen Flüssigkeit. Der flüssige Acrolein-Strom von der Kolonne E (5) wird ebenfalls zum Sumpf der Kolonne F hinzugefügt.
**Konfiguration C (Beispiel 2):** sie entspricht der oben beschriebenen **Prozessoption 2 (****Figur 2****).** Die Input-Parameter für die Simulation betreffend den durch die Kompressoren erreichten Druck, das Zuführdampfverhältnis, das Recyclinggasverhältnis, die Zahl der theoretischen Stufen der Kolonnen und die anderen Parameter des Drucks und der Temperatur, die den Abschnitten A/A1, B, C, D, E und F entsprechen, wurden auf dem jeweils gleichen Niveau wie in den früher genannten Konfigurationen gehalten. Die molaren Flüssigkeitsströme, wie jene der Umpumpungen und der Absorptionsflüssigkeitsphase, wurden in Abhängigkeit von dem Gesamtgasstrom angepasst. Eine Propylenverdampfungseinheit wurde mitbetrachtet.
**Konfiguration D (Vergleichsbeispiel 2):** sie entspricht dem in DE102010064250.9 beschriebenen Prozess. Im Gegensatz zu den früheren Prozessen bzw. Verfahren macht diese Konfiguration keinen Gebrauch von einen Recyclingasstrom. Das inerte Material wird durch eine externe Quelle bereitgestellt. Der erste Prozessblock (Reaktion) dieses Systems besteht aus einer Kompressorstufe für Luft, einer Propylen-Verdampfungseinheit, mindestens einem Rohrbündelreaktor mit integriertem Nachkühler und Dampfgenerator und einem Kompressor für den externen inerten Gasstrom. Auf den Reaktionsblock folgt ein Quench- und Nebenprodukte-Abtrennungsblock ähnlich den dargestellten Kolonnen und peripheren Komponenten um die Kolonnen B und C der früheren Konfigurationen. Der letzte Prozessblock ist analog zu Kolonne F und ihren peripheren Komponenten, die bereits in den Konfigurationen B und C vorhanden sind. Es wurde in diesem Fall festgelegt, dass der Inertgasstrom von einer Verbrennungseinheit stammt, die natürliches Erdgas mit Umgebungsluft als Oxidans verbrennt. Dieses Abgas hat Atmosphärendruck und wird vor der Kompression von 160°C auf 50°C in einem mit Kühlturmwasser betriebenen Wärmeüberträger abgekühlt. Das Gas enthält 4,2 Mol-% O₂ und 12,1 Mol-% H₂O; der Rest entspricht N₂ und CO₂. Die Zuführgaszusammensetzung am Einlass des Reaktors betreffend die Propylen- und Sauerstoffkonzentration ist vergleichbar den in den früheren Konfigurationen verwendeten. Die Bedingungen des Drucks und der Temperatur in den Quench-/Nebenprodukt-Abtrennungs- und Absorptions-/Reaktionsabschnitten waren die gleichen wie in den Konfigurationen B und C. Die molaren Flüssigkeitsströme, wie jene der Umpumpungen wurden in Abhängigkeit von dem Gesamtgasstrom an die Verhältnisse in den Konfigurationen B und C angeglichen.
**Konfiguration E (Vergleichsbeispiel 3):** sie entspricht einem System, in welchem der externe inerte Gasstrom der Konfiguration D durch Dampf ersetzt wird. Danach schließt der Reaktionsprozessblock in dieser Konfiguration nur einen Kompressor für die Luft, eine Propylen-Verdampfungseinheit und mindestens einen Rohrbündelreaktor mit integriertem Nachkühler und Dampfgenerator ein. Nach dem Reaktionsblock treten die Reaktionsgase in eine Quench-/Nebenprodukt-Abtrennungskolonne ein, die zu den Kolonnen B und C und ihren peripheren Komponenten in den vergangenen Konfigurationen analog ist. Der gereinigte Gasstrom wird im nachfolgenden Schritt in das Absorptions-/Reaktionssystem analog zur Kolonne F in den bisherigen Konfigurationen eingespeist. Da es in dieser Konfiguration keinen anderen verfügbaren inerten Gasstrom gibt, wurde Stickstoff in der Kolonne C als Strippmedium verwendet. Die Bedingungen des Drucks und der Temperatur in den Quench-/Nebenprodukt-Abtrennungs- und Absorptions-/Reaktionsabschnitten waren die gleichen wie in den Konfigurationen B und C. Die Flüssigkeitsströme wurden in Abhängigkeit von den Gasströmen angepasst.

Die Ergebnisse der Simulationsbeispiele sind in Tabelle 1 zusammengefasst. Verglichen mit dem standardmäßigen Acrolein-Produktionsprozess (Konfiguration A), weisen beide erfindungsgemäße Prozessaufbauten, die hier vorgestellt wurden (Konfiguration B und C), eine äquivalente Acrolein-Ausbeute auf. Die MMP-Ausbeute ist etwas geringer als im Vergleich mit dem Standardprozess aufgrund der minimalen Menge von MMP, die den Prozess mit dem Abgas verlässt. Aber wegen des Mangels an Recyclinggas, zeigen beide Konfigurationen mit direkter Absorption/Reaktion des produzierten Acroleins in dem MMP (Konfigurationen D und E) eine wesentlich niedrigere Acrolein- und MMP-Ausbeute bezogen auf Propylen. Die Produktausbeuten beeinflussen merklich die Gesamtproduktionskosten von MMP angesichts des relativ hohen Preises von Propylen.

Die erfindungsgemäßen Konfigurationen B und C weisen auch einen nahezu um 50 % geringeren Dampfverbrauch im Vergleich mit dem Standardprozess und etwa 30 % geringer als die Konfiguration E auf. Die Konfiguration D weist überhaupt keinen Dampfverbrauch auf.

Auch den Kaltwasserverbrauch, um Temperaturen auf der Produktseite <35°C zu erreichen betreffend, zeigen die Konfiguration B und C niedrigere Werte als die Konfiguration A. Beide Konfigurationen ohne Destillationsbetrieb (D und E) zeigen noch geringere Verbrauchswerte an gekühltem Wasser.
Von Bedeutung ist auch, dass die Konfigurationen B und C ähnliche Mengen an flüssigen und gasförmigen Abflüssen erzeugen wie der Vergleichs-Prozess (Konfiguration A). Die Konfiguration D andererseits erzeugt nahezu die zweifache Menge Abgas, während die Konfiguration E einen nahezu zweifach höheren Abwasserstrom aber mit einem viel geringeren organischen Gehalt liefert. Im Fall einer Entgiftung dieses Abwassers in einer Verbrennungsanlage sind die Entsorgungskosten dieser Variante deutlich höher als alle anderen hier abgebildeten Verfahren.
Die hier vorgestellten Prozesskonfigurationen A bis E (inklusive der erfindungsgemäßen Prozessoption 1 und Prozessoption 2) wurden mit Hilfe des Programmes Aspen Plus (Version 7.1) und einer validierten Simulation simuliert. Die Packung mit gleicher grundlegender physikalisch-chemischer Eigenschaft wurde für die Simulation von 3 alternativen Prozessen verwendet. Die erfindungsgemäßen Prozessoptionen 1 und 2 (simuliert als Konfiguration B bzw. C) zeigen einen klaren Vorteil gegenüber dem herkömmlichen Produktionsprozess. Auf der einen Seite wird die Zwischenlagerung von großen Volumina an Acrolein vermieden, auf der anderen Seite weist der Prozess einen geringeren Verbrauch an Dampf und Kühlmedien auf. Verglichen mit Prozessen, die auf der direkten Absorption/Reaktion des gasförmigen Acroleins basieren, die entweder von inerten Gasen von einer externen Quelle oder Dampf für die Verdünnung des in den Reaktionsschritt eintretenden Reaktionsgemisches Gebrauch machen, weisen die Prozessierungsoptionen 1 und 2 eine deutlich höhere Produktausbeute und eine geringere Menge an Abfallströmen (Abgas in einem Fall und Abwasser im anderen Fall) auf.

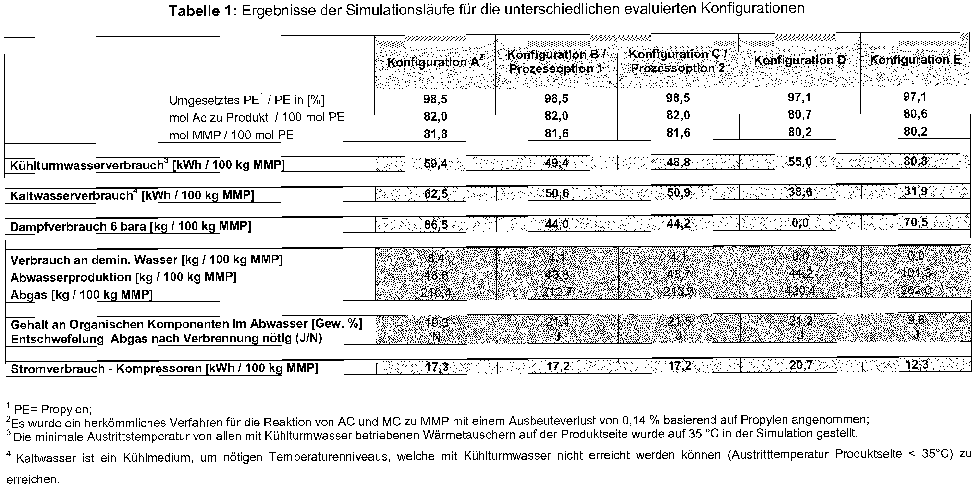

## Patentansprüche

1. Verfahren zur Herstellung von 3-Methylmercaptopropionaldehyd aus Acrolein und Methylmercaptan, bei dem nacheinander die nachfolgenden Schritte durchgeführt werden
A) Gasphasenoxidation von Propylen mit Hilfe von Luft an einem heterogenen Katalysator in Anwesenheit eines Verdünnungsgases
B) Auffangen des Acrolein-haltigen Gasstromes aus A) in einer Quenchstufe zur Abtrennung von Nebenprodukten
C) Rückgewinnen von Rest-Acrolein-Anteilen aus der im unteren Teil der Quenchstufe B) vorhandenen Flüssigkeit durch Strippung
D) Auffangen eines ersten Teiles des Acrolein-haltigen Gasstromes aus der Quenchstufe B) in einer Absorptionsstufe in Gegenwart von Wasser zur Gewinnung einer wässrigen Acrolein-lösung
D1) zumindest teilweise Rückführung des nicht kondensierbaren Gases aus D) als Verdünnungsgas in die Reaktionsstufe A)
E) Destillative Abtrennung des Acroleins aus der wässrigen Acrolein-Lösung aus D) in einer Destillationsstufe
E1) Kondensation des Acrolein-haltigen Destillats aus E) und Zufuhr des Destillats in eine Reaktionsstufe F)
sowie Zufuhr eines weiteren Teiles des Acrolein-haltigen Gasstromes aus der Quenchstufe B) direkt in die Reaktionsstufe F) zur Umsetzung mit Methylmercaptan in Gegenwart von 3-Methylmercaptopropionaldehyd und/oder 3-Methylmercaptopropionaldehyd / Methylmercaptan-Hemithioacetal

2. Verfahren zur Herstellung von 3-Methylmercaptopropionaldehyd aus Acrolein und Methylmercaptan, bei dem nacheinander die nachfolgenden Schritte durchgeführt werden
A) Gasphasenoxidation von Propylen mit Hilfe von Luft an einem heterogenen Katalysator in Anwesenheit eines Verdünnungsgases in einer ersten Reaktionseinheit sowie
A1) gleichzeitige Gasphasenoxidation von Propylen mit Hilfe von Luft an einem heterogenen Katalysator in Anwesenheit eines Verdünnungsgases in einer weiteren Reaktionseinheit
B) Auffangen des Acrolein-haltigen Gasstromes aus A) in einer Quenchstufe unter Kühlung zur Abtrennung von Nebenprodukten
B1) Auffangen es Acrolein-haltigen Gasstromes aus A1) in einer parallelen Quenchstufe zur Abtrennung von Hochsiedern
C) Rückgewinnen von Rest-Acrolein-Anteilen aus der im unteren Teil der Quenchstufe B) vorhandenen Flüssigkeit durch Strippung
D) Auffangen zumindest eines Teiles des Acrolein-haltigen Gasstromes aus der Quenchstufe B) in einer Absorptionsstufe in Gegenwart von Wasser zur Gewinnung einer wässrigen Acrolein-lösung
D1) zumindest teilweise Rückführung des nicht kondensierbaren Gases aus D) als Verdünnungsgas in die Reaktionsstufe A) und A1)
E) Destillative Abtrennung des Acroleins aus der wässrigen Acroleinlösung aus D) in einer Destillationsstufe
E1) Kondensation des Acrolein-haltigen Destillats aus E) und Zufuhr des Destillats in eine Reaktionsstufe F) sowie Zufuhr des Acrolein-haltigen Gasstromes aus der Quenchstufe B1) direkt in die Reaktionsstufe F) zur Umsetzung mit Methylmercaptan in Gegenwart von 3-Methylmercaptopropionaldehyd und/oder 3-Methylmercaptopropionaldehyd / Methylmercaptan-Hemithioacetal

3. Verfahren gemäßen Anspruch 1 oder 2, **dadurch gekennzeichnet, dass** jeweils Schritt A) bzw. Schritt A1) in einem Rohrbündelreaktor durchgeführt werden, dessen Rohre den Katalysator beinhalten.

4. Verfahren gemäß Anspruch 3 **dadurch gekennzeichnet, dass** ein Salzbad zur Kühlung des Rohrbündelreaktors verwendet wird und dieses auf eine Temperatur von 300-400°C gehalten wird.

5. Verfahren gemäß einem der Ansprüche 1 oder 2, **dadurch gekennzeichnet, dass** der Acrolein-haltige Gasstrom aus A) bzw. aus A1) mit einer Temperatur von jeweils 200-280°C in den entsprechenden Schritt B)bzw. B1) gelangt.

6. Verfahren gemäß einem der Ansprüche 1 oder 2, **dadurch gekennzeichnet, dass**.im oberen Drittel der jeweiligen Quenchkolonne B bzw. B1 ein Teilstrom des dort jeweils vorhandenen Kondensats abgeführt wird und gegebenenfalls nach Abkühlen, vorzugsweise auf < 20°C, dem Kopf der jeweiligen Kolonne B bzw. B1 wieder zugeführt wird (oberes Umpumpen).

7. Verfahren gemäß einem der Ansprüche 1 oder 2, **dadurch gekennzeichnet, dass** im Sumpf der jeweiligen Quenchkolonne B bzw. B1 ein Teilstrom der dort jeweils kondensierten Flüssigkeit abgeführt wird und nach Abkühlen dem unteren Drittel der jeweiligen Kolonne B bzw. B1 wieder zugeführt wird (unteres Umpumpen).

8. Verfahren gemäß mindestens einer der Ansprüche 1-7, **dadurch gekennzeichnet, dass** die rückgewonnenen Rest-Acrolein-Anteile aus Schritt C) in Schritt B) zurückgeführt werden.

9. Verfahren gemäß mindestens einer der Ansprüche 1-8, **dadurch gekennzeichnet, dass** die Absorptionsstufe D bei Temperaturen von 1 bis 25°C betrieben wird.

10. Verfahren gemäß mindestens einer der Ansprüche 1-9, **dadurch gekennzeichnet, dass** die Destillationsstufe E bei einem Druck von 0,4 bis 1,2 bara (bar absolut)und bei den sich dabei einstellenden Temperaturen betrieben wird.

11. Verfahren gemäß mindestens einer der Ansprüche 1-10, **dadurch gekennzeichnet, dass** das Acroleinfreie wasserhaltige Sumpfprodukt der Destillation zur Absorptionsstufe D zurückgeführt wird.

12. Verfahren gemäß mindestens einem der Ansprüche 1-11, **dadurch gekennzeichnet, dass** in Schritt F ein basenhaltiger Katalysator verwendet wird.

13. Verfahren gemäß Anspruch 12, **dadurch gekennzeichnet** als basenhaltiger Katalysator ein Amin optional im Gemisch mit einer Säure verwendet wird.

14. Verfahren nach Anspruch 12-13, **dadurch gekennzeichnet, dass** die Base ein ggf. substituiertes N-heterocyclisches Amin oder ein Amin der Formel NR1 R2R3 ist, wobei R1, R2 und R3 gleich oder verschieden sind und unabhängig voneinander H, C1- bis C14-Alkyl, oder C7- bis C14-Aralkyl bedeuten, mit der Maßgabe, dass wenn R1, R2 oder R3 gleich H sind die beiden jeweiligen anderen Reste ungleich H sein müssen.

15. Verfahren nach Anspruch 14, **dadurch gekennzeichnet, dass** die Base Pyridin, alkylsubstituiertes Pyridin, vorzugsweise Picolin oder Lutidin, Trimethylamin, Triethylamin, Tripropylamin, Tributylamin, Tridecylamin, Tridodecylamin oder Dimethylbenzylamin ist.

16. Verfahren nach einem der Ansprüche 12-15, **dadurch gekennzeichnet, dass** die Säure eine Mineralsäure, vorzugsweise Salzsäure, Schwefelsäure, oder Phosphorsäure ist, oder eine organische Säure, vorzugsweise Ameisensäure, Essigsäure, Propionsäure, Milchsäure, Bernsteinsäure, Weinsäure oder Citronensäure.

17. Verfahren nach einem der Ansprüche 1 -16, **dadurch gekennzeichnet, dass** die Reaktionsstufe F bei einem Druck von 1,0 bis 2,5 bara und bei Temperaturen von 50 bis 100 °C betrieben wird.

18. Verfahren nach einem der Ansprüche 1 oder 3-17, **dadurch gekennzeichnet, dass** der weitere Teil des Acrolein-haltigen Gasstroms aus Quenchstufe B), der direkt in die Reaktionsstufe F) geführt wird, einer Menge von 30 - 70 Gew.-%, der Gesamtmenge des Acrolein-haltigen Gases aus B) entspricht.

19. Verfahren gemäß Anspruch 1, **dadurch gekennzeichnet, dass** die Gasphasenoxidation von Propylen in Schritt A parallel in mindestens einem weiteren Reaktor (A1) ausgeführt wird.

20. Verfahren nach Anspruch 19, **dadurch gekennzeichnet, dass** das Auffangen des Acrolein-haltigen Gasstroms aus A1 zur Abtrennung von Nebenprodukten in einer weiteren Quenchstufe B1 geschieht.

## Claims

1. Process for preparing 3-methylmercaptopropionaldehyde from acrolein and methyl mercaptan, in which the following steps are performed successively:
A) gas phase oxidation of propylene with the aid of air over a heterogeneous catalyst in the presence of a diluent gas
B) collecting the acrolein-containing gas stream from A) in a quench stage for removal of by-products
C) recovering the residual acrolein fractions from the liquid present in the lower part of the quench stage B) by stripping
D) collecting a first portion of the acrolein-containing gas stream from the quench stage B) in an absorption stage in the presence of water to obtain an aqueous acrolein solution
D1) at least partly recycling the uncondensable gas from D) as diluent gas into reaction stage A)
E) distillatively removing the acrolein from the aqueous acrolein solution from D) in a distillation stage
E1) condensing the acrolein-containing distillate from E) and supplying the distillate to a reaction stage F)
and feeding a further portion of the acrolein-containing gas stream from the quench stage B) directly into the reaction stage F) for reaction with methyl mercaptan in the presence of 3-methylmercaptopropionaldehyde and/or 3-methylmercaptopropionaldehyde/methyl mercaptan hemithioacetal.

2. Process for preparing 3-methylmercaptopropionaldehyde from acrolein and methyl mercaptan, in which the following steps are performed successively
A) gas phase oxidation of propylene with the aid of air over a heterogeneous catalyst in the presence of a diluent gas in a first reaction unit and
A1) simultaneous gas phase oxidation of propylene with the aid of air over a heterogeneous catalyst in the presence of a diluent gas in a further reaction unit
B) collecting the acrolein-containing gas stream from A) in a quench stage with cooling for removal of by-products
B1) collecting the acrolein-containing gas stream from A1) in a parallel quench stage for removal of high boilers
C) recovering the residual acrolein fractions from the liquid present in the lower part of the quench stage B) by stripping
D) collecting at least a portion of the acrolein-containing gas stream from the quench stage B) in an absorption stage in the presence of water to obtain an aqueous acrolein solution
D1) at least partly recycling the uncondensable gas from D) as diluent gas into reaction stages A) and A1)
E) distillatively removing the acrolein from the aqueous acrolein solution from D) in a distillation stage
E1) condensing the acrolein-containing distillate from E) and supplying the distillate to a reaction stage F)
and feeding the acrolein-containing gas stream from the quench stage B1) directly into the reaction stage F) for reaction with methyl mercaptan in the presence of 3-methylmercaptopropionaldehyde and/or 3-methyl-mercaptopropionaldehyde/methyl mercaptan hemithioacetal.

3. Process according to Claim 1 or 2, **characterized in that** step A) and step A1) are each performed in a shell-and-tube reactor whose tubes comprise the catalyst.

4. Process according to Claim 3, **characterized in that** a salt bath is used to cool the shell-and-tube reactor and it is kept at a temperature of 300-400°C.

5. Process according to either of Claims 1 and 2, **characterized in that** each of the acrolein-containing gas streams from A) and from A1) passes into the corresponding step B) and B1) respectively at a temperature of 200-280°C.

6. Process according to either of Claims 1 and 2, **characterized in that** a substream of the condensate present in the upper third of each quench column B and B1 is removed there and, optionally after cooling, preferably to < 20°C, fed back to the top of the respective column B or B1 (upper pumped circulation).

7. Process according to either of Claims 1 and 2, **characterized in that** a substream of the liquid condensed in the bottom of each quench column B and B1 is removed there and, after cooling, fed back to the lower third of the respective column B or B1 (lower pumped circulation).

8. Process according to at least one of Claims 1-7, **characterized in that** the residual acrolein fractions recovered from step C) are recycled into step B).

9. Process according to at least one of Claims 1-8, **characterized in that** the absorption stage D is operated at temperatures of 1 to 25°C.

10. Process according to at least one of Claims 1-9, **characterized in that** the distillation stage E is operated at a pressure of 0.4 to 1.2 bara (bar absolute) and at the temperatures which are established.

11. Process according to at least one of Claims 1-10, **characterized in that** the acrolein-free aqueous bottom product of the distillation is recycled to the absorption stage D.

12. Process according to at least one of Claims 1-11, **characterized in that** a base-containing catalyst is used in step F.

13. Process according to Claim 12, **characterized in that** the base-containing catalyst used is an amine, optionally in a mixture with an acid.

14. Process according to Claims 12-13, **characterized in that** the base is an optionally substituted N-heterocyclic amine or an amine of the formula NR1R2R3 where R1, R2 and R3 are the same or different and are each independently H, C1-C14-alkyl or C7-C14-aralkyl, with the proviso that, when R1, R2 or R3 is H, the two other radicals in each case must not be H.

15. Process according to Claim 14, **characterized in that** the base is pyridine, alkyl-substituted pyridine, preferably picoline or lutidine, trimethylamine, triethylamine, tripropylamine, tributylamine, tridecylamine, tridodecylamine or dimethylbenzylamine.

16. Process according to any of Claims 12-15, **characterized in that** the acid is a mineral acid, preferably hydrochloric acid, sulphuric acid or phosphoric acid, or an organic acid, preferably formic acid, acetic acid, propionic acid, lactic acid, succinic acid, tartaric acid or citric acid.

17. Process according to any of Claims 1-16, **characterized in that** reaction stage F is conducted at a pressure of 1.0 to 2.5 bara and at temperatures of 50 to 100°C.

18. Process according to any of Claims 1 and 3-17, **characterized in that** the further portion of the acrolein-containing gas stream from quench stage B), which is conducted directly into reaction stage F), corresponds to an amount of 30-70% by weight of the total amount of the acrolein-containing gas from B).

19. Process according to Claim 1, **characterized in that** the gas phase oxidation of propylene in step A is executed in parallel in at least one further reactor (A1).

20. Process according to Claim 19, **characterized in that** the collection of the acrolein-containing gas stream from A1 for removal of by-products is accomplished in a further quench stage B1.

## Revendications

1. Procédé pour la production de 3-méthylmercapto-propionaldéhyde à partir d'acroléine et de méthyl-mercaptan, dans lequel on effectue successivement les étapes suivantes
A) oxydation en phase gazeuse de propylène à l'aide d'air sur un catalyseur hétérogène en présence d'un gaz de dilution
B) collecte du courant de gaz contenant de l'acroléine, provenant de A), dans un étage d'arrêt de réaction pour la séparation de produits secondaires
C) récupération par stripage de fractions d'acroléine résiduelles à partir du liquide présent dans la partie inférieure de l'étage d'arrêt de réaction B)
D) collecte d'une première partie du courant de gaz contenant de l'acroléine, provenant de l'étage d'arrêt de réaction B), dans un étage d'absorption en présence d'eau, pour l'obtention d'une solution aqueuse d'acroléine
D1) recyclage au moins partiel du gaz non condensable provenant de D), en tant que gaz de dilution dans l'étage de réaction A)
E) séparation par distillation de l'acroléine à partir de la solution aqueuse d'acroléine provenant de D), dans un étage de distillation
E1) condensation du distillat contenant de l'acroléine provenant de E) et envoi du distillat dans un étage de réaction F)
ainsi qu'envoi d'une autre partie du courant de gaz contenant de l'acroléine, provenant de l'étage d'arrêt de réaction B), directement dans l'étage de réaction F) pour la réaction avec du méthylmercaptan en présence de 3-méthylmercapto-propionaldéhyde et/ou de 3-méthylmercapto-propionaldéhyde / méthylmercaptan-hémithioacétal.

2. Procédé pour la production de 3-méthylmercapto-propionaldéhyde à partir d'acroléine et de méthylmercaptan, dans lequel on effectue successivement les étapes suivantes
A) oxydation en phase gazeuse de propylène à l'aide d'air sur un catalyseur hétérogène en présence d'un gaz de dilution dans une première unité de réaction ainsi que
A1) oxydation en phase gazeuse simultanée de propylène à l'aide d'air sur un catalyseur hétérogène en présence d'un gaz de dilution dans une autre unité de réaction
B) collecte du courant de gaz contenant de l'acroléine, provenant de A), dans un étage d'arrêt de réaction avec refroidissement, pour la séparation de produits secondaires
B1) collecte du courant de gaz contenant de l'acroléine, provenant de A1), dans un étage d'arrêt de réaction parallèle, pour la séparation de composants à haut point d'ébullition
C) récupération par stripage de fractions d'acroléine résiduelles à partir du liquide présent dans la partie inférieure de l'étage d'arrêt de réaction B)
D) collecte d'au moins une partie du courant de gaz contenant de l'acroléine, provenant de l'étage d'arrêt de réaction B), dans un étage d'absorption en présence d'eau, pour l'obtention d'une solution aqueuse d'acroléine
D1) recyclage au moins partiel du gaz non condensable provenant de D), en tant que gaz de dilution dans l'étage de réaction A) et A1)
E) séparation par distillation de l'acroléine à partir de la solution aqueuse d'acroléine provenant de D), dans un étage de distillation
E1) condensation du distillat contenant de l'acroléine provenant de E) et envoi du distillat dans un étage de réaction F)
ainsi qu'envoi du courant de gaz contenant de l'acroléine, provenant de l'étage d'arrêt de réaction B1), directement dans l'étage de réaction F) pour la réaction avec du méthylmercaptan en présence de 3-méthylmercaptopropionaldéhyde et/ou de 3-méthylmercaptopropionaldéhyde / méthyl-mercaptan-hémithioacétal.

3. Procédé selon la revendication 1 ou 2, **caractérisé en ce que** qu'on effectue l'étape A) ou l'étape A1) chacune dans un réacteur à faisceaux de tubes, dont les tubes comportent le catalyseur.

4. Procédé selon la revendication 3, **caractérisé en ce que** pour le refroidissement du réacteur à faisceaux de tubes on utilise une solution salée et on le maintient à une température de 300-400 °C.

5. Procédé selon l'une quelconque des revendications 1 et 2, **caractérisé en ce que** le courant de gaz contenant de l'acroléine, provenant de A) ou respectivement de A1) arrive dans l'étape B) ou respectivement B1) correspondante à une température dans chaque cas de 200-280 °C.

6. Procédé selon l'une quelconque des revendications 1 et 2, **caractérisé en ce que** dans le tiers supérieur de la colonne d'arrêt de réaction respective B ou B1, un courant partiel du condensat qui y est respectivement présent est évacué et renvoyé, éventuellement après refroidissement, de préférence jusqu'à < 20 °C, à la tête de la colonne respective B ou B1 (recyclage supérieur).

7. Procédé selon l'une quelconque des revendications 1 et 2, **caractérisé en ce que** dans le pied de la colonne d'arrêt de réaction respective B ou B1, un courant partiel du liquide condensé respectivement dans celle-ci est évacué et renvoyé, après refroidissement, au tiers inférieur de la colonne respective B ou B1 (recyclage inférieur).

8. Procédé selon au moins l'une quelconque des revendications 1 à 7, **caractérisé en ce que** les fractions d'acroléine résiduelles récupérées provenant de l'étape C) sont recyclées dans l'étape B).

9. Procédé selon au moins l'une quelconque des revendications 1 à 8, **caractérisé en ce qu'**on fait fonctionner l'étage d'absorption D à des températures de 1 à 25 °C.

10. Procédé selon au moins l'une quelconque des revendications 1 à 9, **caractérisé en ce qu'**on fait fonctionner l'étage de distillation E sous une pression de 0,4 à 1,2 bara (bar absolu) et aux températures qui s'ajustent dans ces conditions.

11. Procédé selon au moins l'une quelconque des revendications 1 à 10, **caractérisé en ce qu'**on renvoie à l'étage d'absorption D le produit de pied de la distillation, exempt d'acroléine, contenant de l'eau,

12. Procédé selon au moins l'une quelconque des revendications 1 à 11, **caractérisé en ce que** dans l'étape F on utilise un catalyseur contenant une base.

13. Procédé selon la revendication 12, **caractérisé en ce que** comme catalyseur contenant une base, on utilise une amine en option en mélange avec un acide.

14. Procédé selon la revendication 12-13, **caractérisé en ce que** la base est une amine N-hétérocyclique éventuellement substituée ou une amine de formule NR1R2R3, où R1, R2 et R3 sont identiques ou différents et représentent chacun indépendamment H, un groupe alkyle en C₁-C₁₄ ou aralkyle en C₇-C₁₄, étant entendu que lorsque R1, R2 ou R3 est H, les deux autres radicaux respectifs doivent être différents de H.

15. Procédé selon la revendication 14, **caractérisé en ce que** la base est la pyridine, une pyridine substitués par alkyle, de préférence la picoline ou la lutidine, la triméthylamine, la triéthylamine, la tripropylamine, la tributylamine, la tridécylamine, la tridodécylamine ou la diméthylbenzylamine.

16. Procédé selon l'une quelconque des revendications 12 à 15, **caractérisé en ce que** l'acide est un acide minéral, de préférence l'acide chlorhydrique, l'acide sulfurique ou l'acide phosphorique, ou un acide organique, de préférence l'acide formique, l'acide acétique, l'acide propionique, l'acide lactique, l'acide succinique, l'acide d-tartrique ou l'acide citrique.

17. Procédé selon l'une quelconque des revendications 1 à 16, **caractérisé en ce qu'**on fait fonctionner l'étage de réaction F sous une pression de 1,0 à 2,5 barsa et à des températures de 50 à 100 °C.

18. Procédé selon l'une quelconque des revendications 1 ou 3 à 17, **caractérisé en ce que** l'autre partie du courant de gaz contenant de l'acroléine, provenant de l'étage d'arrêt de réaction B), qui est envoyée directement dans l'étage de réaction F), correspond à une quantité de 30 - 70 % en poids de la quantité totale du gaz contenant de l'acroléine, provenant de B).

19. Procédé selon la revendication 1, **caractérisé en ce que** l'oxydation en phase gazeuse de propylène dans l'étape A est effectuée parallèlement dans au moins un autre réacteur (A1).

20. Procédé selon la revendication 19, **caractérisé en ce que** la collecte du courant de gaz contenant de l'acroléine, provenant de A1, pour la séparation des produits secondaires, a lieu dans un autre étage d'arrêt de réaction B1.
